(19) Europäisches Patentamt
European Patent Office
Office européen des brevets

(11) Veröffentlichungsnummer: **0 011 824**
**B1**

(12) # EUROPÄISCHE PATENTSCHRIFT

(45) Veröffentlichungstag der Patentschrift: **14.10.81**

(51) Int. Cl.³: **C 07 D 405/04,** D 06 L 3/12 // C07D307/85

(21) Anmeldenummer: **79104642.8**

(22) Anmeldetag: **22.11.79**

(54) **Benzofuranyl-benzimidazole, Verfahren zu ihrer Herstellung sowie ihre Verwendung zum optischen Aufhellen von organischen Materialien.**

(30) Priorität: **05.12.78 DE 2852531**

(43) Veröffentlichungstag der Anmeldung: **11.06.80 Patentblatt 80/12**

(45) Bekanntmachung des Hinweises auf die Patenterteilung: **14.10.81 Patentblatt 81/41**

(84) Benannte Vertragsstaaten: **CH DE FR GB IT**

(56) Entgegenhaltungen: **DE-A-2 733 156**

(73) Patentinhaber: **BAYER AG, Zentralbereich Patente, Marken und Lizenzen, D-5090 Leverkusen 1, Bayerwerk (DE)**

(72) Erfinder: **Bremen, Josef, Dr., Am Kettnersbusch 1e, D-5090 Leverkusen 3 (DE)**
Erfinder: **Wehling, Bernhard, Dr., Andreas-Gryphius-Strasse 26, D-5000 Köln 80 (DE)**

**EP 0 011 824 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Benzofuranyl-benzimidazole, Verfahren zu ihrer Herstellung sowie ihre Verwendung zum optischen Aufhellen von organischen Materialien

Die vorliegende Erfindung betrifft Benzofuranylbenzimidazole der Formel

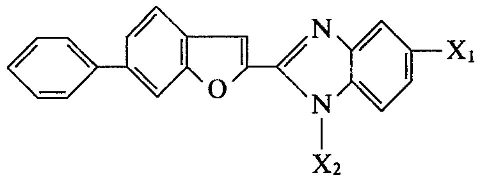

(I)

worin

$X_1$ Wasserstoff, $C_1$—$C_4$-Alkyl, Chlor oder $C_1$—$C_4$-Alkylsulfonyl,
$X_2$ Wasserstoff, $C_1$—$C_4$-Alkyl, Phenyl oder Benzyl bedeuten,

sowie deren Protonierungs- und Quaternierungsprodukte.

Die neuen Benzofuran-benzimidazole können nach verschiedenen Verfahren hergestellt werden. Eine Methode ist dadurch gekennzeichnet, daß man Verbindungen der Formel

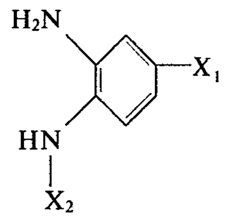

(V)

in der $X_1$ und $X_2$ die unter Formel (I) angegebene Bedeutung haben, in an sich bekannter Weise (vgl. US-PS 3 940 417 und 4 009 994) mit der Carbonsäure der Formel

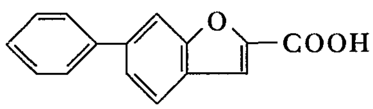

(IV)

oder einem ihrer funktionellen Derivate der Carboxylgruppe umsetzt. Vorzugsweise führt man diese Umsetzung in Gegenwart saurer Kondensationsmittel, wie z. B. Essigsäure, Chlorwasserstoff, Borsäure, Zinkchlorid, Polyphosphorsäure, Phosphorsäure oder p-Toluolsulfonsäure durch. Die erhaltenen Verbindungen werden gegebenenfalls in bekannter Weise quaterniert oder mit einer Säure protoniert.

Zu den nicht quaternierten Verbindungen der Formel (I) gelangt man auch, indem man am Imidazolring unsubstituierte Verbindungen der Formel (I), worin $X_1$ Wasserstoff bedeutet, mit Alkylierungsmitteln in Gegenwart von basisch wirkenden Verbindungen nach bekannten Verfahren umsetzt.

Unter funktionellen Derivaten der Carbonsäure der Formel (IV) sind deren Salze, Halogenide, Ester, Amide, Iminoäther sowie Nitrile zu verstehen.

Die Herstellung der Protonierungs- bzw. Quaternierungsprodukte kann auf üblichem Wege, vorzugsweise in einem Lösungsmittel, vorgenommen werden, wobei es zweckmäßig ist, mindestens ein Moläquivalent des Protonierungs- bzw. Quaternierungsmittels einzusetzen.

Sofern es gewünscht ist, Verbindungen der Formel (I) herzustellen, welche mit Alkylresten quaterniert sind, so verwendet man als Alkylierungsmittel vorzugsweise Dialkylsulfate wie Dimethyl- und Diäthylsulfat, Alkylhalogenide wie Methylchlorid, Äthyl-, Propyl- und Butyliodid oder -bromid, Allylchlorid oder -bromid, Crotylchlorid oder -bromid sowie Alkylbenzolsulfonate wie p-Methyl-, Äthyl- oder Chlorbenzolsulfonat. Wenn es gewünscht ist, Verbindungen der Formel (I) herzustellen, die mit einem Benzylrest quaterniert sind, so verwendet man für die Benzylierung vorzugsweise Benzylhalogenide wie Benzylchlorid. Weitere Quaternierungsmittel sind z. B. $BrCH_2CH_2OH$, $BrCH_2CHOHCH_3$, Halogenessigsäurederivate wie $ClCH_2CO_2CH_2CH_3$, $BrCH_2COOH$, $BrCH_2COOCH_3$, $ClCH_2CN$, $ClCH_2CONH_2$, $ClCH_2CONHCH_3$ und $ClCH_2CON(CH_3)_2$ sowie Äthylenoxid oder Propylenoxid in Gegenwart geeigneter Anionen wie z. B. der Ameisen-, Essig- oder Milchsäure.

Zur Herstellung protonierter Verbindungen der Formel (I) verwendet man als Protonierungsmittel insbesondere Mineralsäuren. Geeignet sind prinzipiell alle starken bis mittelstarken organischen Säuren oder Mineralsäuren, wobei die Anionen durch doppelte Umsetzung ausgetauscht werden können.

Als Lösungsmittel, in denen die Protonierung bzw. Quaternierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe; Halogenkohlenwasserstoffe, Nitrobenzol, Alkanole und offene oder cyclische Äther; Ketone, Fettsäureamide, Sulfoxide und Carbonsäureester. Dabei wird z. B. bei Temperaturen von 60 bis 180°C, vorzugsweise 90 bis 140°C gearbeitet. Zuweilen ist es auch vorteilhaft, überschüssiges Alkylierungsmittel als Lösungsmittel zu verwenden.

Alternativ können die Verbindungen der Formel (I) auch hergestellt werden, indem man ein o-Nitroanilin der Formel

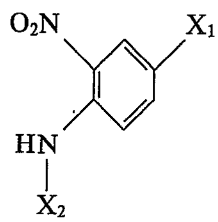

(VI)

worin

$X_1-X_2$ die unter Formel (I) angegebene Bedeutung haben, mit einer Verbindung der Formel (IV) oder einem funktionellen Derivat davon acyliert, die Nitrogruppe in saurem Medium reduziert, z. B. mit Stannochlorid/Salzsäure und gleichzeitig den Ringschluß zum Imidazolring herbeiführt.

Die Ausgangsprodukte der Formel (V) werden in an sich bekannter Weise durch Umsetzung von o-Chlor-nitrobenzolderivaten mit primären Aminen oder Ammoniak zu den entsprechenden substituierten o-Nitroanilinen und Reduktion der letzteren, z. B. mittels katalytischer Hydrierung, hergestellt (vgl. BE-PS 595 327, DE-OS 2 239 614 und DE-OS 1 522 412).

Die Ausgangsverbindungen der Formel (IV) können hergestellt werden:

1. durch Cumarilsäureumlagerung (vgl. Krauch-Kunz 3. Aufl. Reaktionen der organischen Chemie, S. 449) aus dem Cumarinderivat der Formel

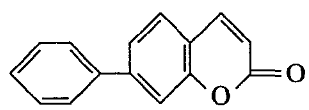

(VII)

2. durch Umsetzung von Verbindungen der Formel

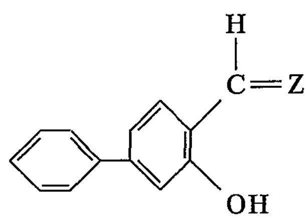

(VIII)

worin

Z = 0 oder $N-R_1$, wobei
$R_1$ einen gegebenenfalls substituierten Phenylrest bedeutet,

mit Halogenmalonestern der Formel

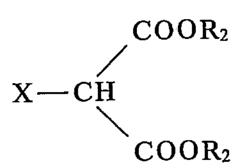

(IX)

worin

X Chlor, Brom oder Jod, insbesondere Brom und
$R_2$ eine Alkylgruppe, insbesondere Methyl oder Äthyl bedeuten,

in an sich bekannter Weise (vgl. Spagnolo, J. Chem. Soc. Perkin Trans. 1972, S. 1), wobei zunächst die Verbindung der Formel

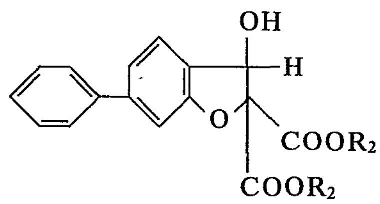

(X)

entsteht, die dann durch Verseifung, Dehydratisierung und Decarboxylierung in die Verbindung der Formel (IV) übergeführt wird; hierbei wird die erste Reaktionsstufe zweckmäßigerweise in polaren Lösungsmitteln, wie Alkoholen, Ketonen, Fettsäureamiden sowie Sulfoxiden in Gegenwart säurebindender Substanzen, bei 50 bis 120°C und die zweite Reaktionsstufe zweckmäßigerweise in einem polaren Lösungsmittel, insbesondere einem Alkohol, in Gegenwart einer starken Base, wie Natriumhydroxid oder Kaliumhydroxid, oder der wäßrigen Lösung einer starken Base bei 60 bis 150°C durchgeführt, oder

3. durch Umsetzung der Verbindungen der Formel (VIII) mit Halogenessigestern der Formel

$$Y-CH_2-COOR_3 \qquad (XI)$$

worin

Y Chlor, Brom oder Jod, insbesondere Chlor oder Brom und
$R_3$ eine Alkylgruppe bedeuten,

in an sich bekannter Weise (vgl. Bull. Soc. Chim. France 1971, S. 4329 bis 4331), wobei die Umsetzung zweckmäßigerweise in Lösung in Gegenwart säurebindender Substanzen durchgeführt wird. Geeignete Lösungsmittel sind z. B. Wasser, Alkohole oder Carbonsäurederivate, wie Acetonitril, Dimethylformamid oder N-Methylpyrrolidon. Auch Gemische der genannten Lösungsmittel sind geeignet.

Die erfindungsgemäßen Verbindungen der Formel (I) zeigen in gelöstem oder feinverteiltem Zustand eine ausgeprägte Fluoreszenz. Sie eignen sich zum optischen Aufhellen der verschiedensten hochmolekularen Materialien, vor allem zum optischen Aufhellen von Fasern, Fäden, Geweben, Gewirken oder Folien synthetischer Herkunft, z. B. aus Polyestern, wie Polyterephthalsäureglykolestern, Polyamiden, wie Polymere auf Basis von Hexamethylendiaminadipat oder Caprolactam, Celluloseestern, wie Cellulose $2^1/_2$-Acetat und Cellulosetriacetat, und insbesondere aus Polyacrylnitril.

Erfindungsgemäß optisch aufgehelltes Fasermaterial weist ein gefälliges, rein weißes, blauviolett bis blaustichig fluoreszierendes Aussehen auf; in hellen Farbtönen gefärbtes und erfindungsgemäß weißgetöntes derartiges Material zeichnet sich durch reinen Farbton aus.

Waschflotten, die Benzofurane der Formel (I) enthalten, verleihen beim Waschen den damit behandelten Textilfasern, beispielsweise synthetischen Polyamid-, Polyester- und Celluloseesterfasern, insbesondere aber Polyacrylnitrilfasern, einen brillanten Aspekt im Tageslicht. Die erzielten Aufhellungen weisen hohe Echtheit auf und sind insbesondere lichtecht und waschbeständig.

Im Vergleich zu nächstvergleichbaren bekannten Aufhellern gemäß DE-A 2 733 156, die an Stelle des erfindungsgemäßen isolierten 6-Phenylrestes einen ankondensierten Benzolrest im Benzofuran-Grundgerüst tragen, zeichnen sich die quaternierten Verbindungen der Formel (I) durch eine wesentlich verbesserte Aufhellwirkung der Polyacrylnitrilfärbungen aus.

## Beispiel 1

In eine Lösung von 4,1 g N-Methyl-o-phenylendiamin in 60 ml Pyridin werden bei Raumtemperatur innerhalb von 5 Minuten 7,7 g 6-Phenylbenzofuran-2-carbonsäurechlorid eingetragen. Hierbei steigt die Temperatur auf 35–40°C an. Es wird 15 Minuten nachgerührt und dann 6 Stunden bei 80–85°C gerührt. Es wird auf Raumtemperatur abgekühlt und in ca. 350 ml 10%ige Salzsäure gegossen. Es wird 30 Minuten bei Raumtemperatur gerührt und, wenn nötig, mit einigen Millilitern 10%iger Salzsäure kongosauer gestellt. Es wird ein kristalliner Niederschlag erhalten. Man saugt ab und wäscht mehrmals mit 5%iger Salzsäure nach.

Das so erhaltene Acylierungsprodukt wird noch feucht in 150 ml Glykolmonomethyläther bei 80°C suspendiert. Man versetzt mit 10 ml konzentrierter Salzsäure, wobei sich das Produkt augenblicklich auflöst. Es wird 4 Stunden unter Rückfluß gerührt. Nach Abkühlen auf Raumtemperatur wird die

Lösung mit ca. 300 ml Wasser verdünnt. Die erhaltene Suspension wird mit Triäthylamin neutralisiert. Es wird 10 Minuten nachgerührt und dann von dem erhaltenen kristallinen Niederschlag abgesaugt.

Nach Umkristallisieren aus Isopropanol werden 6,1 g (63% der Theorie) des Benzimidazols der Formel

(101)

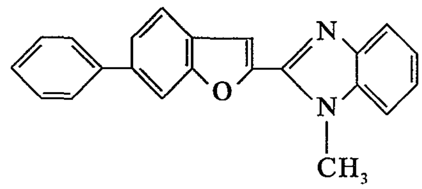

in Form weißer Kristalle erhalten, welche in Lösung rotviolett fluoreszieren.

Verwendet man anstelle von N-Methyl-o-phenylendiamin die äquivalente Menge 5-Methyl-2-methylaminoanilin bzw. 5-Methylsulfonyl-2-methylaminoanilin und verfährt im übrigen wie vorstehend beschrieben, so erhält man die Benzimidazole der Formel

(102)

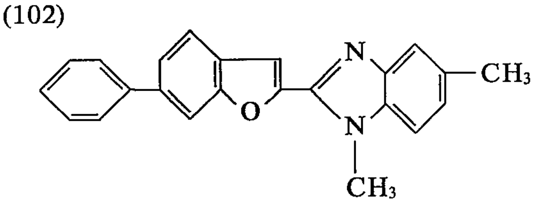

Fluoreszenzfarbe in Lösung: rot-violett bzw.

(103)

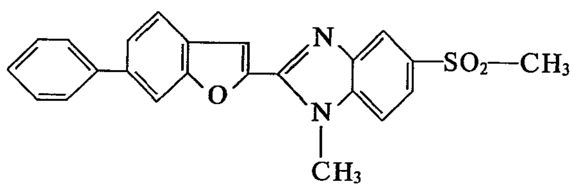

Fluoreszenzfarbe in Lösung: blau-violett.

Auf analoge Weise gelangt man zu den in Tabelle 1 aufgeführten Verbindungen.

Tabelle I

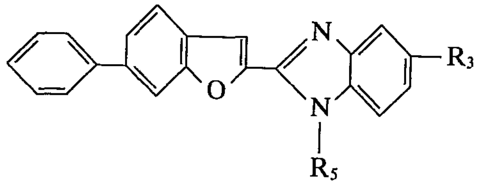

| Verbindung | $R_3$ | $R_5$ | Fluoreszenzfarbe in Lösung |
|---|---|---|---|
| 104 | H | $CH_2—C_6H_5$ | rot-violett |
| 105 | H | 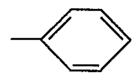 | rot-violett |
| 106 | $CH_3$ | $—CH_2—C_6H_5$ | rot-violett |
| 107 | $CH_3$ | 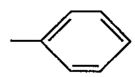 | rot-violett |
| 108 | $SO_2—CH_3$ | $—CH_2—C_6H_5$ | blau-violett |
| 109 | $SO_2—CH_3$ | $C_2H_5$ | blau-violett |
| 110 | Cl | $CH_3$ | blau-violett |
| 111 | Cl | $CH_2—C_6H_5$ | blau-violett |
| 112 | Cl | 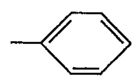 | blau-violett |

Das als Ausgangsprodukt verwendete 6-Phenylbenzofuran-2-carbonsäurechlorid wird wie folgt hergestellt:

Eine Suspension von 20 g 4-Phenylsalicylaldehyd, 15 g Chloressigsäureäthylester, 28,2 g wasserfreiem Kaliumcarbonat und 1 g Kaliumjodid in 200 ml Dimethylformamid wird zunächst 3 Stunden bei 70°C dann 1 Stunde unter Rückfluß gerührt. Hierauf wird das Lösungsmittel im Wasserstrahlvakuum abdestilliert. Der Rückstand wird in einer Lösung aus 200 ml Äthanol und 40 g 50%iger Kalilauge 6 Stunden unter Rückfluß erhitzt. Das Gemisch wird auf 1000 ml Wasser gegossen und mit Tonsil in der Siedehitze geklärt. Das Filtrat wird bei Raumtemperatur mit konzentrierter Salzsäure kongosauer gestellt. Der Niederschlag wird abgesaugt, neutral gewaschen und bei 80°C getrocknet. Es werden 18 g (77% der Theorie) 6-Phenylbenzofuran-2-carbonsäure vom Schmelzpunkt 238°C erhalten.

Eine aus Chlorbenzol umkristallisierte Probe schmilzt bei 260°C (Lit.: 262°C).

16,0 g der so hergestellten 6-Phenyl-benzofuran-2-carbonsäure werden mit 15 g Thionylchlorid und 0,2 ml Dimethylformamid in 150 ml Toluol 2 Stunden unter Rückfluß erhitzt. Hierauf werden das überschüssige Thionylchlorid und das Lösungsmittel im Vakuum abdestilliert und der Rückstand aus Ligroin (Siedebereich 90—110°C) umkristallisiert. Es werden 14,8 g (85,8% der Theorie) 6-Phenyl-benzofuran-2-carbonsäurechlorid vom Schmelzpunkt 105—107°C erhalten.

## Beispiel 2

Eine Lösung von 2,0 g der Verbindung der Formel (101) und 0,9 g Dimethylsulfat in 100 ml Toluol wird 6 Stunden bei 120°C gerührt. Es wird auf 0°C abgekühlt, wobei das quaternäre Benzimidazoliumsalz ausfällt. Man saugt ab, wäscht mit Toluol nach und trocknet. Es werden 2,6 g (93,6% der Theorie) des quaternären Benzofuranyl-benzimidazolium-Salzes der Formel

(201)

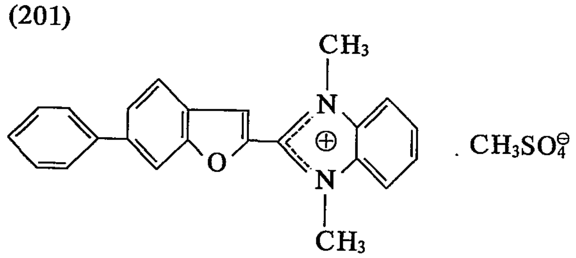

in Form weißer Kristalle erhalten.

Die Verbindung löst sich in Wasser mit violetter Fluoreszenz am Tageslicht und ist sehr gut geeignet zum Aufhellen von Polyacrylnitrilfasern.

Verwendet man anstelle der Verbindung der Formel (101) die gleichen Mengen der Verbindungen der Formel (102) bzw. (103) und verfährt im übrigen wie vorstehend beschrieben, so erhält man die quaternären Salze der Formeln

(202)

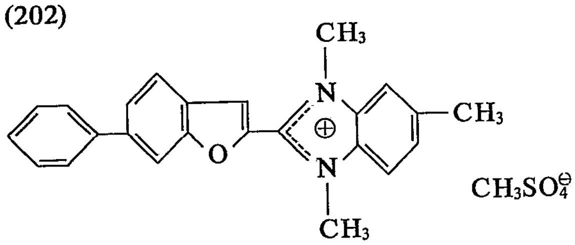

bzw.

(203)

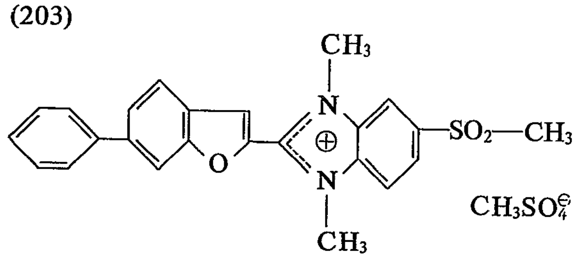

Die Verbindungen lösen sich in Wasser mit blau-violetter Fluoreszenz und sind vorzüglich geeignet zum Aufhellen von Polyacrylnitrilfasern.

Auf analoge Weise gelangt man zu den in Tabelle 2 aufgeführten Verbindungen:

Tabelle 2

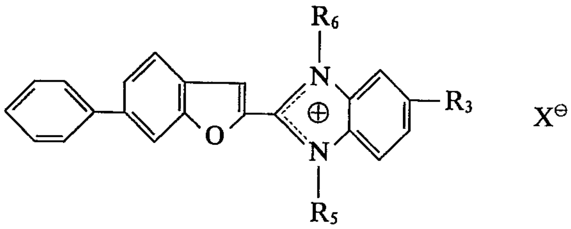

| Verbindung | $R_3$ | $R_5$ | $R_6$ | X | Fluoreszenzfarbe in Lösung |
|---|---|---|---|---|---|
| 204 | H | $CH_2—C_6H_5$ | $CH_3$ | $CH_3SO_4^{\ominus}$ | blau-violett |
| 205 | H | 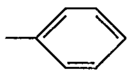 | $CH_3$ | $CH_3SO_4^{\ominus}$ | blau-violett |
| 206 | $CH_3$ | $CH_2—C_6H_5$ | $CH_3$ | $CH_3SO_4^{\ominus}$ | blau-violett |
| 207 | $CH_3$ | 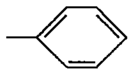 | $CH_3$ | $CH_3SO_4^{\ominus}$ | blau-violett |
| 208 | $SO_2—CH_3$ | $-CH_2-C_6H_5$ | $CH_3$ | $CH_3SO_4^{\ominus}$ | blau |
| 209 | $SO_2—CH_3$ | $—CH_3$ | $CH_2—C_6H_5$ | $Cl^{\ominus}$ | blau |
| 210 | $SO_2—CH_3$ | $—CH_3$ | $CH_2—COOC_2H_5$ | $Cl^{\ominus}$ | blau |
| 211 | $SO_2—CH_3$ | $C_2H_5$ | $CH_3$ | $CH_3SO_4^{\ominus}$ | blau |
| 212 | Cl | $CH_3$ | $CH_3$ | $CH_3SO_4^{\ominus}$ | blau |
| 213 | Cl | 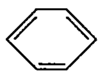 | $CH_3$ | $CH_3SO_4^{\ominus}$ | blau |

Beispiel 3

Polyacrylnitrilfasern werden im Flottenverhältnis 1 : 40 30 Minuten kochend mit einer Färbeflotte behandelt, die 0,2% der Verbindung der Formel (202), 8% Na-chlorit, 4% Na-nitrat, 4% Chloritstabilisator (alle Prozentangaben bezogen auf das Textilmaterial) enthält und mit Ameisensäure auf pH 3,5 gestellt ist. Nach dem Spülen und Trocknen erhält man ein sehr gut und brillant aufgehelltes Polyacrylnitrilgewebe.

Ähnliche Ergebnisse erhält man, wenn man wie oben beschrieben verfährt, jedoch die Verbindungen der Formel (201) oder (203) einsetzt.

**Patentansprüche**

1. Benzofuranyl-benzimidazole der Formel

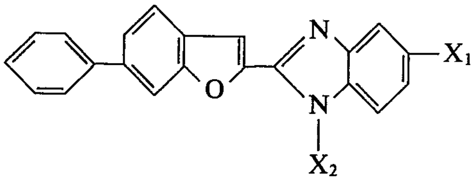

(I)

worin

$X_1$ Wasserstoff, $C_1-C_4$-Alkyl, Chlor oder $C_1-C_4$-Alkylsulfonyl,
$X_2$ Wasserstoff, $C_1-C_4$-Alkyl, Phenyl oder Benzyl bedeuten,

sowie deren Protonierungs- und Quaternierungsprodukte.

2. Benzofuranyl-benzimidazol gemäß Anspruch 1 der Formel

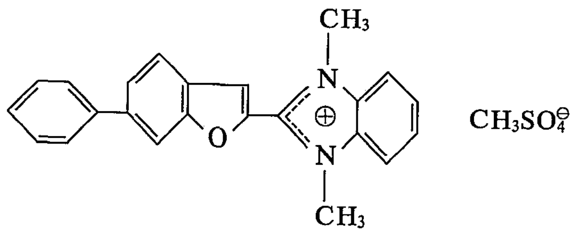

3. Benzofuranyl-benzimidazol gemäß Anspruch 1 der Formel

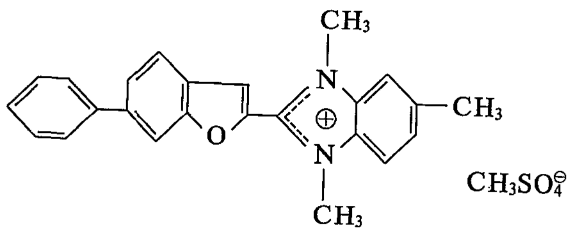

4. Benzofuranyl-benzimidazol gemäß Anspruch 1 der Formel

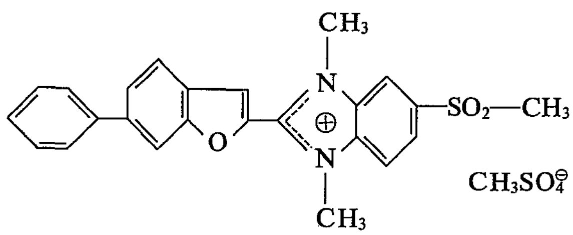

5. Verfahren zur Herstellung von Benzofuranyl-benzimidazolen gemäß Anspruch 1, dadurch gekennzeichnet, daß man Verbindungen der Formel

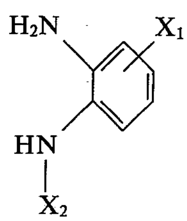

in der $X_1$ und $X_2$ die in Anspruch 1 genannte Bedeutung haben, mit der Carbonsäure der Formel

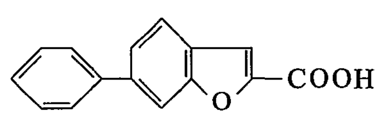

oder deren funktionellen Derivaten in an sich bekannter Weise ringschließend umsetzt und die erhaltenen Verbindungen gegebenenfalls in bekannter Weise quaterniert oder protoniert.

6. Verfahren zum Weißtönen von organischen Materialien, dadurch gekennzeichnet, daß man Benzimidazolyl-benzofurane gemäß Anspruch 1 verwendet.

**Claims**

1. Benzofuranyl-benzimidazoles of the formula

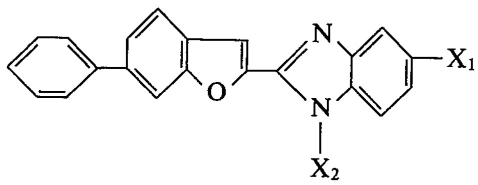

(I)

in which

$X_1$ denotes hydrogen, $C_1-C_4$ alkyl, chlorine or $C_1-C_4$ alkylsulphonyl,
$X_2$ denotes hydrogen, $C_1-C_4$ alkyl, phenyl or benzyl,

and protonation and quaternisation products thereof.

2. Benzofuranyl-benzimidazole according to Claim 1 of the formula

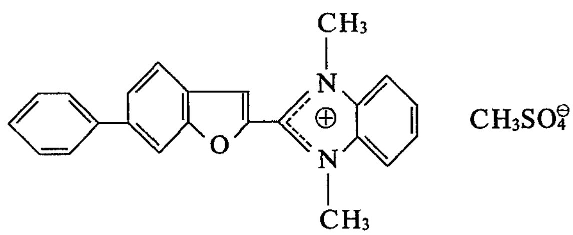

3. Benzofuranyl-benzimidazole according to Claim 1 of the formula

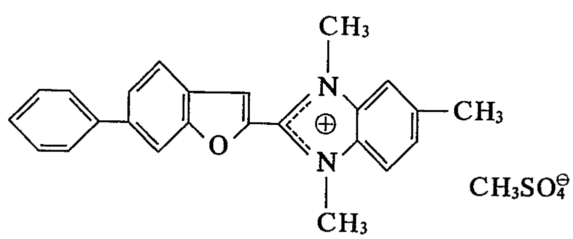

4. Benzofuranyl-benzimidazole according to Claim 1 of the formula

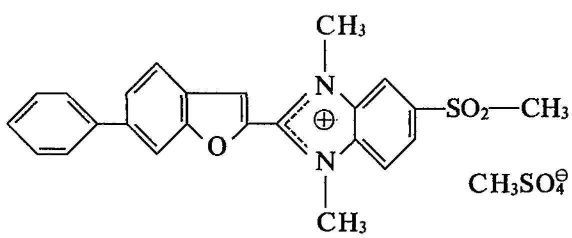

5. Process for the preparation of benzofuranyl-benzimidazoles according to Claim 1, characterised in that compounds of the formula

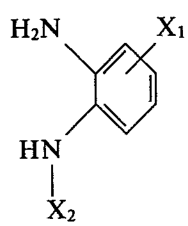

in which $X_1$ and $X_2$ have the meaning mentioned in Claim 1, are cyclised with the carboxylic acid of the formula

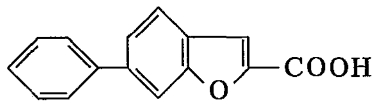

or functional derivatives thereof in a manner which is itself known and the resulting compounds are optionally quaternised or protonated in a known manner.

6. Process for brightening organic materials, characterised in that benzimidazolyl-benzofuranes according to Claim 1 are used.

**Revendications**

1. Benzofurannyl-benzimidazoles de formule

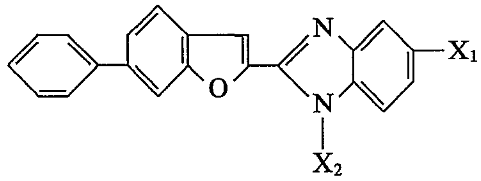

(I)

dans laquelle

$X_1$ représente l'hydrogène, un alkyle en $C_1-C_4$, le chlore ou un alkylsulfonyle en $C_1-C_4$,
$X_2$ représente l'hydrogène, un alkyle en $C_1-C_4$, un phényle ou un benzyle,

ainsi que leurs produits de protonisation et de quaternisation.

2. Benzofurannyl-benzimidazole selon la revendication 1 de formule

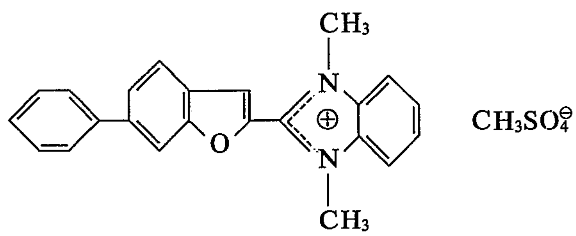

3. Benzofurannyl-benzimidazole selon la revendication 1 de formule

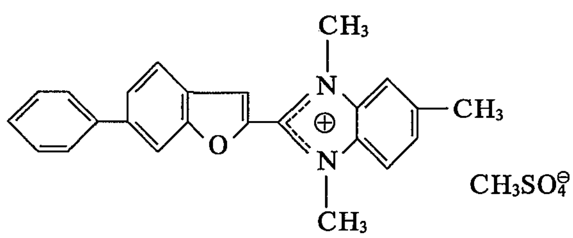

4. Benzofurannyl-benzimidazole selon la revendication 1 de formule

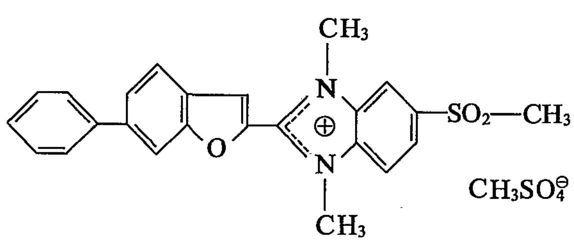

5. Procédé pour la préparation de benzofurannyl-benzimidazoles selon la revendication 1, caractérisé en ce que l'on fait réagir les composés de formule

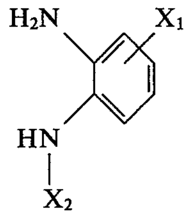

dans laquelle $X_1$ et $X_2$ ont les significations indiquées à la revendication 1, avec l'acide carboxylique de formule

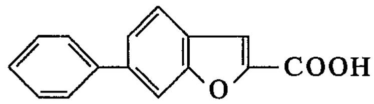

ou un de ses dérivés fonctionnels du groupe carboxyle avec cyclisation de manière connue et on quaternise ou protonise éventuellement les composés obtenus.

6. Procédé pour l'azurage de matières organiques caractérisé en ce que l'on utilise des benzimidazolyl-benzofurannes selon la revendication 1.